# EUROPÄISCHE PATENTSCHRIFT

(11) **EP 1 293 163 B1**
(45) Veröffentlichungstag und Bekanntmachung des Hinweises auf die Patenterteilung: **20.07.2005**
(21) Anmeldenummer: 02019481.7
(22) Anmeldetag: 30.08.2002
(51) Int. Cl.: A61B 5/20

(54) **Vorrichtung für eine urologische Untersuchung eines Patienten mit einem Miktionssitz**
Device for urological examination of a patient with a urinary chair
Appareil d'examen urologique d'un patient comportant un siège-urinoir

(30) Priorität: 12.09.2001 DE 10144975
(43) Veröffentlichungstag der Anmeldung: 19.03.2003
(73) Patentinhaber: SIEMENS AKTIENGESELLSCHAFT, 80333 München (DE)
(72) Erfinder: Winkelmann, Günther, 91074 Herzogenaurach (DE)

(56) Entgegenhaltungen:
- EP-A- 0 208 886
- DE-A- 4 406 493
- US-A- 1 961 459

## Beschreibung

Die Erfindung betrifft eine Vorrichtung für eine urologische Untersuchung eines Patienten mit einem Miktionssitz, mit einem Trägerteil und mit einer eine Drehachse aufweisenden Halterung, mit welcher der Miktionssitz an dem Trägerteil befestigt ist, und welche eine Drehung des Miktionssitzes relativ zu dem Trägerteil erlaubt.

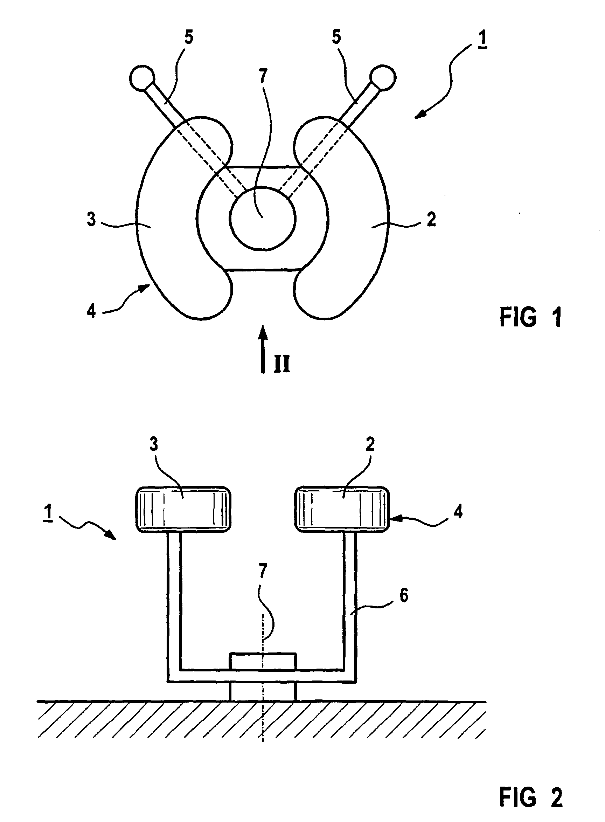

In den schematischen Fig. 1 und 2 ist eine derartige Vorrichtung gezeigt. Die Vorrichtung 1, welche in Fig. 1 in einer Ansicht von oben und in Fig. 2 in einer Ansicht in Richtung des Pfeiles II aus Fig. 1 dargestellt ist, umfasst einen zwei Sitzpolster 2, 3 aufweisenden Miktionssitz 4, ein Trägerteil 5 und eine U-förmig ausgeführte Halterung 6 für den Miktionssitz 4. Die Halterung 6 weist eine Drehachse 7 auf, um welche die Halterung 6 drehbar ist und welche, wie insbesondere aus Fig. 1 zu erkennen ist, mittig in Bezug auf den Miktionssitz 4 angeordnet ist.

Diese Ausbildung der Halterung hat den Nachteil, dass der Raum unterhalb des Miktionssitzes durch die Halterung verbaut ist. Daher können urodynamische Messeinrichtungen, welche im Rahmen von urologischen Untersuchungen unterhalb des Miktionssitzes platziert werden müssen, nur unter Schwierigkeiten dort angeordnet werden. Die urodynamischen Messeinrichtungen haben dabei in der Regel immer Kontakt mit der Halterung oder dem Miktionssitz, weshalb Messergebnisse beeinflusst werden können.

Aus der DE 44 06 493 C2 ist ein Miktionssitz mit Beinhaltern zur Unterstützung der Oberschenkel eines Patienten für ein urologisches Röntgenuntersuchungsgerät bekannt, wobei unterhalb des Miktionssitzes Freiraum vorhanden ist. Der Miktionssitz kann an einer Patientenlagerungsplatte des Röntgenuntersuchungsgerätes derart befestigt werden, dass er durch Umklappen der Patientenlagerungsplatte mit dieser zusammen um eine Drehachse außermittig in Bezug auf den Miktionssitz um 90° vertikal drehbar ist.

Der Erfindung liegt daher die Aufgabe zugrunde, eine Vorrichtung der eingangs genannten Art derart auszuführen, dass der Miktionssitz auf einfache Weise relativ zu dem Trägerteil verstellbar ist und der Raum unterhalb des Miktionssitzes praktisch frei zugänglich ist.

Nach der Erfindung wird diese Aufgabe gelöst durch eine Vorrichtung für eine urologische Untersuchung eines Patienten mit einem eine Längsachse aufweisenden Miktionssitz, mit einem Trägerteil und mit einer wenigstens eine Drehachse aufweisenden Halterung, mit welcher Halterung der Miktionssitz an dem Trägerteil befestigt ist, und welche Halterung eine Verstellung des Miktionssitzes relativ zu dem Trägerteil erlaubt, wobei die eine Drehachse der Halterung außermittig in Bezug auf den Miktionssitz angeordnet ist, dadurch gekennzeichnet, dass die Halterung eine zweite Drehachse aufweist, welche die Längsachse des Miktionssitzes schneidet. Durch die außermittige Anordnung der Drehachse der Halterung wird unterhalb des Miktionssitzes Freiraum geschaffen, so dass urodynamische Messeinrichtungen problemlos unterhalb des Miktionssitzes frei positioniert werde können, ohne mit dem Miktionssitz oder der Halterung in Berührung zu kommen.

Die Vorrichtung ist vorzugsweise derart ausgebildet, dass die eine Drehachse der Halterung zumindest in einer Ausgangsstellung des Miktionssitzes relativ zu dem Trägerteil, in der noch keine Verstellung des Miktionssitzes um die eine Drehachse erfolgt ist, windschief zur Längsachse des Miktionssitzes angeordnet ist. Die Längsachse des Miktionssitzes und die eine Drehachse schneiden sich demnach nicht. Nach einer Variante der Erfindung ist die eine Drehachse der Halterung dabei wenigstens im Wesentlichen rechtwinklig zu der Längsachse des Miktionssitzes angeordnet ist.

Die Erfindung sieht außerdem vor, dass die Halterung eine zweite Drehachse aufweist, welche die Längsachse des Miktionssitzes schneidet. Nach einer vorteilhaften Variante der Erfindung schneidet die zweite Drehachse der Halterung die Längsachse des Miktionssitzes wenigstens im Wesentlichen rechtwinklig.

Durch die erfindungsgemäße Ausgestaltung der Vorrichtung kann der Miktionssitz wenigstens um 90° relativ zu dem Trägerteil verdreht werden, wobei in jeder Stellung des Miktionssitzes relativ zum Trägerteil Freiraum zwischen dem Miktionssitz und dem Boden vorhanden ist, um urodynamische Messeinrichtungen unterhalb des Miktionssitzes platzieren zu können.

Ein Ausführungsbeispiel der Erfindung ist in den beigefügten Zeichnungen dargestellt. Es zeigen:
- Fig. 1: eine Vorrichtung für eine urologische Untersuchung eines Patienten mit einem Miktionssitz nach dem Stand der Technik,
- Fig. 2: eine Ansicht der Vorrichtung in Richtung des Pfeiles II aus Fig. 1,
- Fig. 3: eine Ansicht einer erfindungsgemäßen Vorrichtung für eine urologische Untersuchung eines Patienten mit einem Miktionssitz, und
- Fig. 4, 5: Ansichten der Vorrichtung aus Fig. 3 in Richtung des Pfeiles III aus Fig. 3 bei zwei verschiedenen Stellungen des Miktionssitzes.

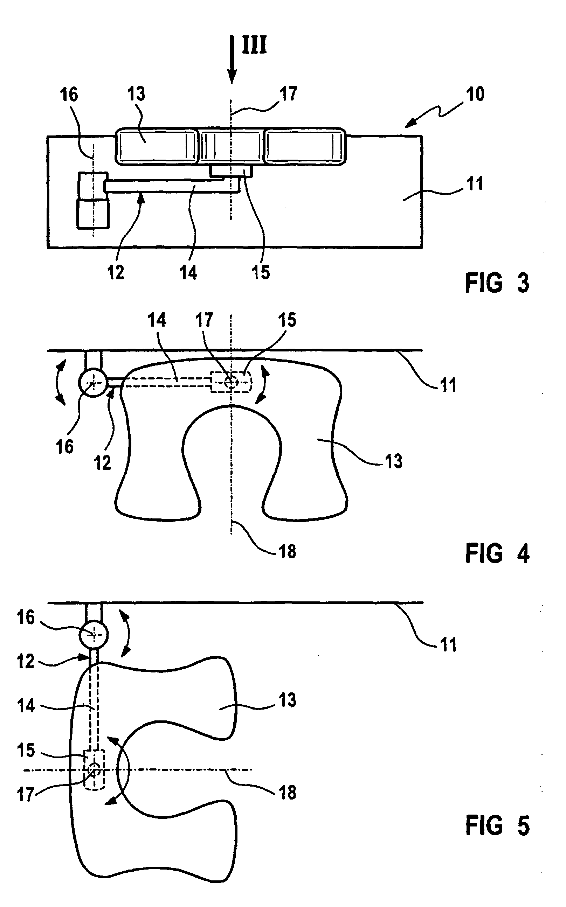

In Fig. 3 ist eine erfindungsgemäße Vorrichtung 10 für eine urologische Untersuchung eines in den Figuren nicht dargestellten Patienten gezeigt. Die Vorrichtung 10 weist ein Trägerteil 11, eine Halterung 12 und einen Miktionssitz 13 auf. Der Miktionssitz 13 ist mit der Halterung 12 an dem Trägerteil 11 befestigt. Die Halterung 12 weist einen Ausleger 14, eine Platte 15, eine erste Drehachse 16 und eine zweite Drehachse 17 auf. An der mit dem Ausleger 14 verbundenen Platte 15 ist der Miktionssitz 13 befestigt. Der Ausleger 14 ist dabei um die erste Drehachse 16 der Halterung 12 und die Platte 15 um die zweite Drehachse 17 der Halterung 12 relativ zu dem Ausleger 14 drehbar.

Wie insbesondere der Fig. 4 entnommen werden kann, welche eine Ansicht der Vorrichtung 10 in Richtung des Pfeiles III aus Fig. 3 zeigt, ist die erste Drehachse 16 der Halterung 12 außermittig in Bezug auf den Miktionssitz 13, welcher eine Längsachse 18 aufweist, angeordnet. In der in den Fig. 3 und 4 gezeigten Ausgangsstellung des Miktionssitzes 13 relativ zu dem Trägerteil 11, schneidet die erste Drehachse 16 der Halterung 12 die Längsachse 18 des Miktionssitzes 13 also nicht, sondern ist zu dieser versetzt bzw. windschief angeordnet. Die erste Drehachse 16 der Halterung 12 und die Längsachse 18 des Miktionssitzes stehen dabei unabhängig von Verstellungen des Miktionssitzes 13 um die Drehachsen 16, 17 wenigstens im Wesentlichen rechtwinklig zueinander.

Die zweite von der ersten verschiedene Drehachse 17 schneidet die Längsachse 18 des Miktionssitzes, wobei die beiden Achsen wenigstens im Wesentlichen einen rechten Winkel einschließen. Die zweite Drehachse 17 verläuft dabei wenigstens im Wesentlichen parallel zur ersten Drehachse 16.

In Fig. 5 ist ein Verstellung des Miktionssitzes 13 um die Drehachsen 16, 17 relativ zu dem Trägerteil 11 gezeigt. Es ist zu erkennen, dass der Miktionssitz 13 mit der Halterung 12 um wenigstens 90° relativ zum dem Trägerteil gedreht werden kann.

Mit der erfindungsgemäßen Vorrichtung 10 ist unabhängig von der Stellung des Miktionssitzes 13 relativ zu dem Trägerteil 11 unterhalb des Miktionssitzes 13 Freiraum geschaffen, um urodynamische Messgeräte für urologische Untersuchungen problemlos ohne Kontakt zum Miktionssitz 13 oder der Halterung 12 platzieren zu können.

## Patentansprüche

1. Vorrichtung für eine urologische Untersuchung eines Patienten mit einem eine Längsachse (18) aufweisenden Miktionssitz (13), mit einem Trägerteil (11) und mit einer wenigstens eine Drehachse (16, 17) aufweisenden Halterung (12), mit welcher Halterung (12) der Miktionssitz (13) an dem Trägerteil (11) befestigt ist, und welche Halterung (12) eine Verstellung des Miktionssitzes (13) relativ zu dem Trägerteil (11) erlaubt, wobei die eine Drehachse (16) der Halterung (12) außermittig in Bezug auf den Miktionssitz (13) angeordnet ist,
**dadurch gekennzeichnet, dass**
die Halterung (12) eine zweite Drehachse (17) aufweist, welche die Längsachse (18) des Miktionssitzes (13) schneidet.

2. Vorrichtung nach Anspruch 1, bei der die eine Drehachse (16) der Halterung (12) in einer Ausgangsstellung des Miktionssitzes (13) relativ zu dem Trägerteil (11), in der noch keine Verstellung des Miktionssitzes (13) um die eine Drehachse (16) erfolgt ist, windschief zur Längsachse (18) des Miktionssitzes (13) angeordnet ist.

3. Vorrichtung nach Anspruch 1 oder 2, bei der die eine Drehachse (16) der Halterung (12) wenigstens im Wesentlichen rechtwinklig zu der Längsachse (18) des Miktionssitzes (13) angeordnet ist.

4. Vorrichtung nach einem der Ansprüche 1 bis 3, bei der die zweite Drehachse (17) der Halterung (12) die Längsachse (18) des Miktionssitzes (13) wenigstens im Wesentlichen rechtwinklig schneidet.

## Claims

1. Device for a urological examination of a patient with a urinary chair comprising a longitudinal axis (18), with a carrier piece (11) and with a mounting support (12) comprising at least one axis of rotation (16, 17), said mounting support (12) fixing the urinary chair (13) to the carrier piece (11), and allowing an adjustment of the urinary chair (13) in relation to the carrier piece (11), whereby the one axis of rotation (16) of the mounting support (12) is arranged excentrically in relation to the urinary chair (13),
**characterised in that**
the mounting support (12) has a second axis of rotation (17) which intersects the longitudinal axis (18) of the urinary chair (13).

2. Device according to Claim 1, in which the one axis of rotation (16) of the mounting support (12) in a starting position of the urinary chair (13), in which no adjustment of the urinary chair (13) takes place about an axis of rotation (16), is arranged skew to the longitudinal axis (18) of the urinary chair (13) relative to the support piece (11).

3. Device according to Claim 1 or 2, in which the one axis of rotation (16) of the mounting support (12) is arranged at least essentially at right angles to the longitudinal axis (18) of the urinary chair (13).

4. Device according to one of the Claims 1 to 3, in which the second axis of rotation (17) of the mounting support (12) at least essentially intersects the longitudinal axis (18) of the urinary chair (13) at right angles.

## Revendications

1. Appareil d'examen urologique d'un patient, comprenant un siège-urinoir (13) ayant un axe (18) longitudinal, une partie (11) de support et une fixation (12) ayant au moins un axe (16, 17) de rotation, fixation (12) par laquelle le siège-urinoir (13) est fixé à la partie (11) de support et fixation (12), qui permet de déplacer le siège-urinoir (13) par rapport à la partie (11) de support, l'axe (16) de rotation de la fixation (12) étant disposé de manière excentrée par rapport au siège-urinoir (13), **caractérisé en ce que** la fixation (12) comporte un deuxième axe (17) de rotation, qui coupe l'axe (18) longitudinal du siège-urinoir (13).

2. Appareil suivant la revendication 1, dans lequel l'axe (16) de rotation de la fixation (12) est, dans une position initiale du siège-urinoir (13) par rapport à la partie (11) de support, dans laquelle il n'a pas encore été effectué de déplacement du siège-urinoir (13) autour de l'axe (16) de rotation, disposé de façon à ne pas couper l'axe (18) longitudinal du siège-urinoir (13).

3. Appareil suivant la revendication 1 ou 2, dans lequel l'axe (16) de rotation de la fixation (12) est au moins sensiblement perpendiculaire à l'axe (18) longitudinal du siège-urinoir (13).

4. Appareil suivant l'une des revendications 1 à 3, dans lequel le deuxième axe (17) de rotation de la fixation (12) coupe l'axe (18) longitudinal du siège-urinoir (13) au moins sensiblement à angle droit.
